# EUROPEAN PATENT APPLICATION

(11) **EP 2 298 326 A2**
(43) Date of publication of application: **23.03.2011**
(21) Application number: 10182500.8
(22) Date of filing: 14.01.2000
(51) Int. Cl.: A61K 31/715

(54) **Administration of an anti-endotoxin drug by intravenous infusion**

(30) Priority: 14.01.1999 US 116202 P
(62) Divisional of application: 07019438.6
(71) Applicant: Eisai R&D Management Co., Ltd., Tokyo 112-8088 (JP)
(72) Inventor: Rossignol, Daniel P., Mahwah, NJ 07430 (US); Lynn, Melvyn, East Brunswick, NJ 08816 (US); Kerns, William D., Harvard, MA 01451 (US)
(74) Representative: Müller, Christian Stefan Gerd

(57) **Abstract**

The invention provides methods for administering an anti-endotoxin drug, E5564, by intravenous infusion. The methods can be used for treating conditions such as endotoxemia, sepsis, and septic shock.

## Description

### Background of the Invention

This invention relates to a regimen of administration of an anti-endotoxin drug.

Since the 1930's, the increasing use of immunosuppressive therapy and invasive devices as well as the increased incidence of antibiotic resistance in bacteria have lead to a gradual rise in the occurrence of sepsis and septic shock. Currently, the estimated incidences in the U.S. of sepsis and septic shock are 400,000 and 200,000 patients/year, respectively. This results in about 100,000 fatalities/year, making septic shock the most common non-coronary cause of death in the hospital Intensive Care Unit (ICU). Currently, ICU therapy for septic shock is limited to antibiotic therapy, cardiovascular resuscitation, vasopressor/ionotrope therapy, and ventilatory support. This ICU care can cost up to $1,500/day and average a total of $13,000 to $30,000 per patient. Clearly, any therapy that can reduce the morbidity and therefore the cost of care in sepsis/septic shock will be of great value.

It is likely that antibiotics themselves can worsen morbidity associated with sepsis; their bactericidal action can result in the release of endotoxin from Gram negative bacteria, which are believed to induce many pathophysiological events such as fever, shock, disseminated intravascular coagulation (DIC), and hypotension. Consequently, medicines for the treatment of Gram negative sepsis have been desired for some time, especially drugs capable of blocking endotoxin or cytokines derived from endotoxin-mediated cellular stimulation. To this end, various strategies for treatment have included antibodies against LPS or cytokines, such as TNF-α and interleukin-1. For various reasons, these approaches have failed.

While endotoxin itself is a highly heterogenous molecule, the expression of many of the toxic properties of endotoxin is attributed to a highly conserved hydrophobic lipid A portion. An effective drug that acts as an antagonist to this conserved structure is known as E5564 (also known as compound 1287 and SGEA). This drug is described as compound 1 in U.S. Patent No. 5,681,824, which is hereby incorporated by reference. E5564 has the formula:
(α-D-Glucopyranose, 3-*O*-decyl-2-deoxy-6-*O*-[2-deoxy-3-*O*-[(3R)-3-methoxydecyl)-6-*O*-methyl-2-[[(11Z)-1-oxo-11-octadecenyl)amino]-4-*O*-phosphono-β-D-glucopyranosyl]-2-[(1,3-dioxotetradecyl)amino]-, 1-(dihydrogen phosphate),
which can be provided as a tetrasodium salt. E5564 has a molecular weight of 1401.6.

### Summary of the Invention

We have discovered that administration of E5564 by continuous infusion over a relatively long period of time overcomes an unexpectedly short pharmacodynamic half-life of the drug, which surprisingly has been observed even though E5564 demonstrates a long pharmacokinetic half-life in circulation in the blood.

Accordingly, the invention features methods of treating patients suffering from medical conditions amenable to treatment with E5564. Examples of such conditions include endotoxemia (*e.g.,* surgery-related endotoxemia), sepsis, septic shock, HIV infection, and immunological disorders, such as graft-versus-host disease and allograft rejection.

In the methods of the invention, E5564 is administered to patients by intravenous infusion over a period of 12-100, preferably 60-80, and more preferably 72 hours. Activity in the ICU is often hectic, and minor variations in the time period of infusion of the drug are included within the scope of the invention.

Preferably, the infusion dosage rate is 0.001-0.5 mg/kg body weight/hour, more preferably 0.01-0.2 mg/kg/hour, and most preferably 0.03-0.1 mg/kg/hour. The infusion of E5564 can, if desired, be preceded by a bolus injection of E5564; preferably, such a bolus injection is given at a dosage of 0.001-0.5 mg/kg. Preferably, the total amount of E5564 administered to a patient is 50-600 mg of drug, more preferably 150-500 mg, by infusion over a period of 60-80 hours.

The total dosage of drug is advantageously quite high, providing a maximum therapeutic effect, but, surprisingly, is not accompanied by unacceptable toxicity. In particular, as is described further below, it has been found that, although injected or infused E5564 remains present in the blood for a relatively long period of time (*i.e.,* E5564 has a relatively long pharmacokinetic half-life), the period during which it is active (*i.e.,* its pharmacodynamic half-life) is relatively short. Thus, it is advantageous to administer the drug by continuous infusion over a prolonged period of time.

The invention also includes the use of E5564, in the dosages set forth above, in the treatment of the conditions set forth above, as well as the use of E5564, in the dosages set forth above, in the preparation of medicaments for treating these conditions.

It is unexpected that such prolonged administration is possible, because a related, three- to ten-fold less active anti-endotoxin compound, B531 (U.S. Patent No. 5,530,113, which is hereby incorporated by reference), could not be safely administered to patients in such a manner, due to its toxicity. Surprisingly, E5564 is about twenty-fold less toxic than B531, and thus can be administered at relatively high levels, for relatively long periods of time, according to the methods of the invention. Thus, the methods of the invention provide significant therapeutic benefits, with acceptably low toxicity. An additional advantage of the methods of the invention is that they are easily carried out, as many of the patients treated according to the methods of the invention already have intravenous lines inserted, as part of their treatment in the ICU.

The invention, in particular, relates to the following:
1. Use of Compound E5564 for the preparation of a pharmaceutical composition for treating a patient suffering from a medical condition amenable to treatment with said compound, wherein the pharmaceutical composition is to be administered to said patient by intravenous infusion over a period of 12-100 hours.
2. The use of item 1, wherein infusion is carried out over a period of 60-80 hours.
3. The use of item 2, wherein infusion is carried out over a period of 72 hours.
4. The use of item 1, wherein the infusion/dosage rate is 0.001-0.5 mg/kg body weight/hour.
5. The use of item 4, wherein the infusion/dosage rate is 0.01-0.2 mg/kg body weight/hour.
6. The use of item 5, wherein the infusion/dosage rate is 0.03-0.1 mg/kg body weight/hour.
7. The use of item 1, wherein said infusion is preceded by a bolus injection of said compound.
8. The use of item 7, wherein said bolus injection is at a dosage of 0.001-0.5 mg/kg body weight.
9. The use of item 1, wherein the total amount of said compound administered to the patient is 50-600 mg of drug.
10. The use of item 9, wherein the amount of drug administered is 150-500 mg, over a period of 60-80 hours.
11. The use of item 1, wherein the medical condition is selected from the group consisting of endotoxemia, sepsis, septic shock, HIV infection, immunological disorders, graft-versus-host-disease and allograft rejection.

### Other features and advantages of the invention will be apparent from the following detailed description, the drawings, and the claims.

### Brief Description of the Drawings

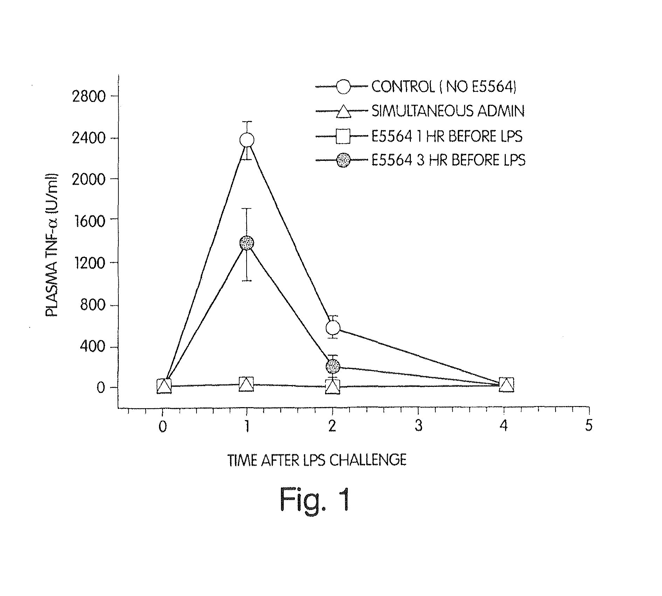

Figure 1 is a graph showing the anti-endotoxin activity of E5564 after a single bolus injection. LPS endotoxin (300 ng/kg) was injected intravenously into untreated dogs (o) or simultaneously with 0.1 mg/kg E5564 (Δ) one hour after E5564 administration (□) or three hours after E5564 administration (•). Blood was drawn and analyzed for TNF-α concentration by bioassay, as is described in the Appendix, below. Each value represents the mean ± S.E.M. of four animals.

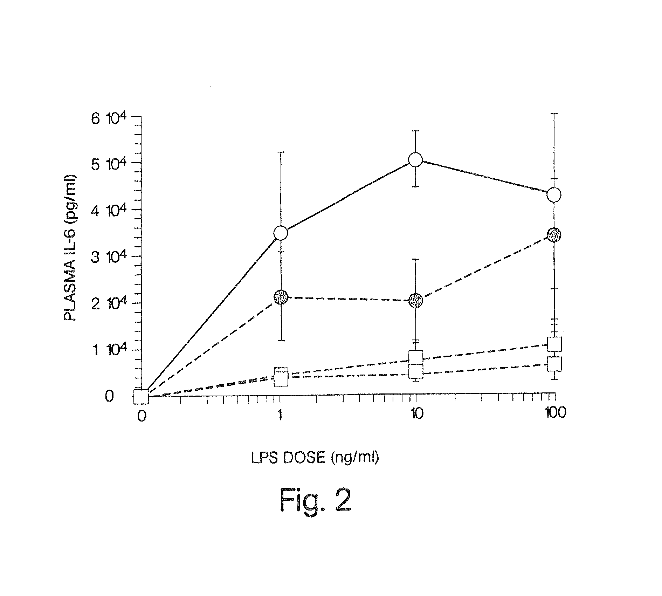

Figure 2 is a graph showing induction of IL-6 in dog blood ex vivo; dose response to LPS in pre-dose blood samples. Blood samples from male dogs #101 (ο) and #201 (•), and female dogs #151 (□) and #251 (■), were drawn prior to dosing, treated with the indicated amount of LPS for 3 hours, and assayed for release of IL-6 (see Appendix).

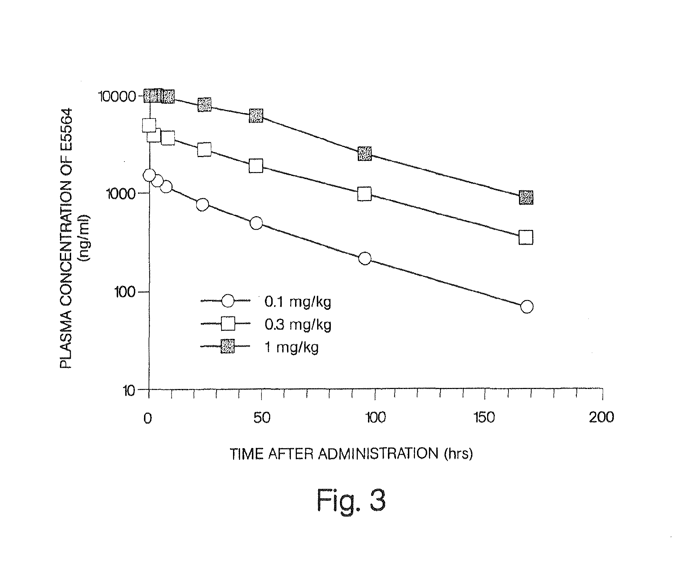

Figure 3 is a graph showing the plasma levels of E5564 after a single bolus injection. After bolus administration of 0. 1 mg/kg E5564 (o), 0.3 mg/kg E5564 (□), and 1 mg/kg E5564 (■), blood was drawn and analyzed for E5564 concentration by extraction and analysis by HPLC. Each value represents the mean ± S.E.M. of three animals. No drug was detectable in samples drawn prior to dosing.

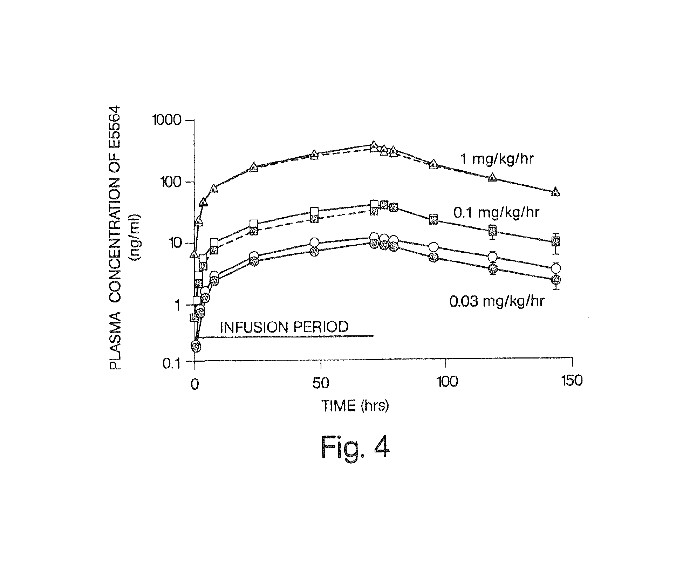

Figure 4 is a graph showing the plasma levels of E5564 during and after 72 hours of intravenous infusion. Plasma levels of E5564 were determined during and after 72 hours of intravenous infusion of 0.03 mg/kg/hr E5564 (o,•), 0.1 mg/kg/hr E5564 (□,■), and 1 mg/kg/hr E5564 (Δ,▲) into female (closed symbols) or male (open symbols) beagle dogs. At the indicated times, blood was drawn and analyzed for E5564 concentration by extraction and analysis by HPLC. Each value represents the meant S.E.M. of three animals. No drug was detectable in samples drawn prior to dosing.

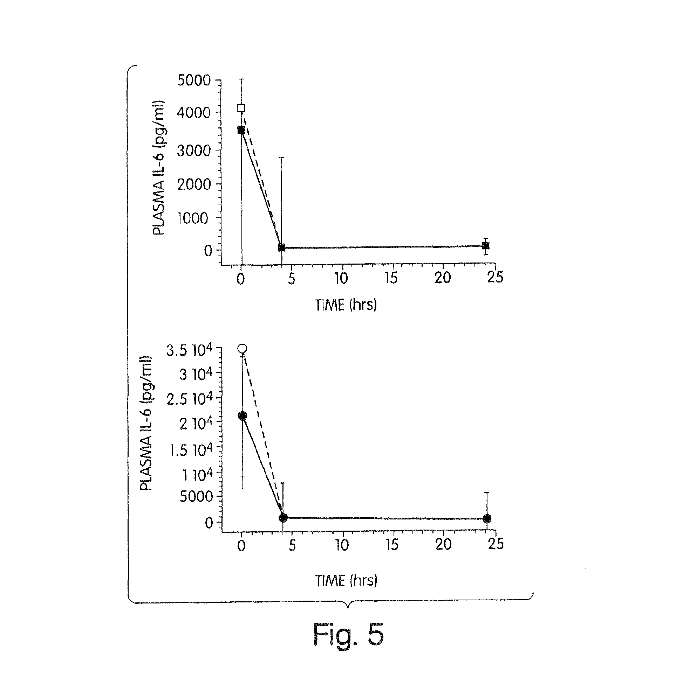

Figure 5 is a pair of graphs showing *ex vivo* analysis of active E5564 during intravenous infusion. Activity of E5564 was determined during intravenous infusion of 0.24 mg/kg/hr E5564 (□,o) or 2.4 mg/kg/hr E5564 (■,•) into female (upper panel) or male (lower panel) beagle dogs. At the indicated times, blood was drawn and analyzed for E5564 activity by adding 1 ng/ml LPS, incubating for three hours at 37°C, and assaying the plasma fraction for IL-6 by bioassay, as is described in the Appendix. Each value represents the mean ± standard deviation of samples assayed in duplicate from each animal. The zero hour sample was taken prior to infusion.

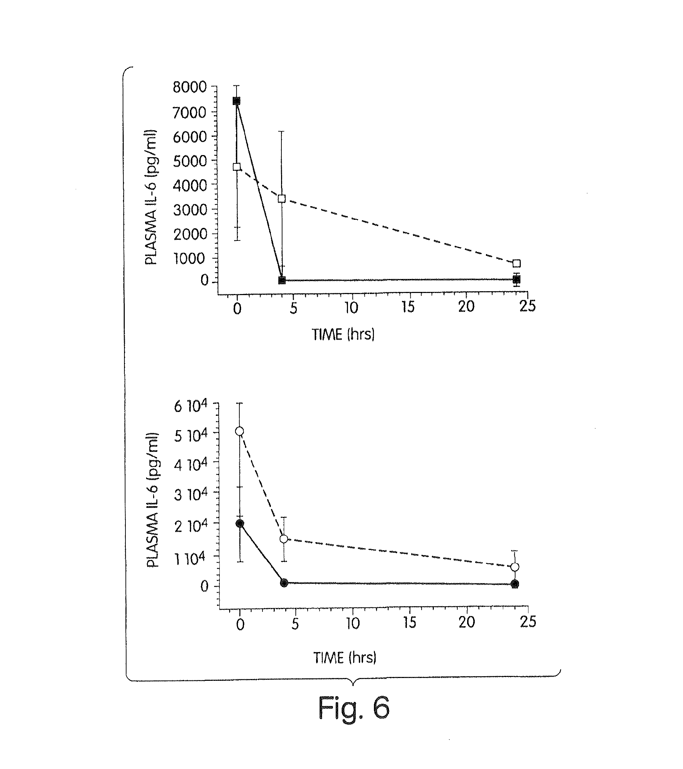

Figure 6 is a pair of graphs showing *ex vivo* analysis of active E5564 during intravenous infusion. Activity of E5564 was determined during intravenous infusion of 0.24 mg/kg/hr E5564 (□,ο) or 2.4 mg/kg/hr E5564 (■,•) into female (upper panel) or male (lower panel) beagle dogs. At the indicated times, blood was drawn and analyzed for E5564 activity by adding 10 ng/ml LPS, incubating for three hours at 37°C, and assaying the plasma fraction for IL-6 by bioassay, as is described in the Appendix. Each value represents the mean ± standard deviation of samples assayed in duplicate from each animal. The zero hour sample was taken prior to infusion.

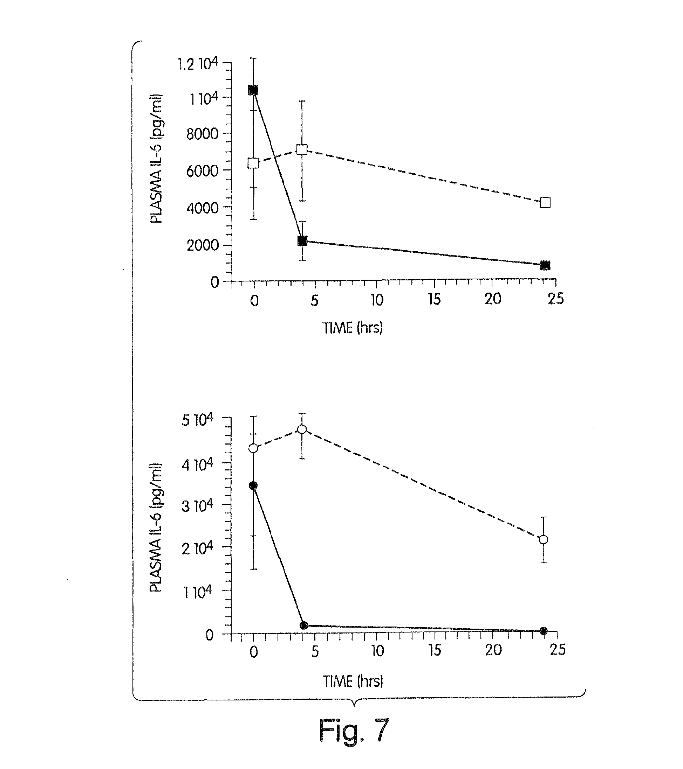

Figure 7 is a pair of graphs showing *ex vivo* analysis of active E5564 during intravenous infusion. Activity of E5564 was determined during intravenous infusion of 0.24 mg/kg/hr E5564 (□,ο) or 2.4 mg/kg/hr E5564 (■,•) into female (upper panel) or male (lower panel) beagle dogs. At the indicated times, blood was drawn and analyzed for E5564 activity by adding 100 ng/ml LPS, incubating for three hours at 37°C, and assaying the plasma fraction for IL-6 by bioassay, as is described in the Appendix. Each value represents the mean ± standard deviation of samples assayed in duplicate from each animal. The zero hour sample was taken prior to infusion.

### Detailed Description

As is noted above, we have discovered that administration of E5564 by continuous infusion over a relatively long period of time overcomes an unexpectedly short pharmacodynamic half-life of the drug, which surprisingly has been observed even though E5564 demonstrates a long pharmacokinetic half-life in circulation in the blood. The methods of the invention, as well as experimental data related to these methods, are described further, as follows.

### Analysis of anti-endotoxin drug activity

Many of the signs and symptoms of sepsis can be mimicked *in vivo* by administration of endotoxin to an animal model system. The physiological effects of endotoxin can vary depending on dose, route of administration, and species tested, but generally include symptoms such as elevated temperature (fever), hypotension, changes in cellular composition of blood (decreased white blood cells, etc.), and elevation of proinflammatory cytokines, such as TNF-α and IL-6, and some anti-inflammatory cytokines. The activity of a drug designed to antagonize the effects of endotoxin can be tested in animal model studies by determining if it blocks any or all of these physiological markers of endotoxin activity.

In general, the candidate antagonist is administered to a test species of animal, and an appropriate dose of endotoxin (lipopolysaccharide (LPS)) is administered to test the ability of the candidate antagonist to block the effects of endotoxin. Some of the experiments described below use an *in vivo* challenge of LPS given intravenously both during and after intravenous infusion of E5564. Activity of an antagonist can also be assayed ex vivo by removing blood samples from animals treated with the candidate antagonist and testing that blood to determine if the drug is active and/or present in sufficient quantities to inhibit cellular activation by LPS. In both assays, activity of the antagonist is quantitated by analysis of the cytokines induced by LPS administration. In addition, other physiological symptoms of endotoxin poisoning can be used as readouts of activity. Studies described herein use TNF-α and/or IL-6 as readouts of cellular activation, but a variety of other cytokines and cellular mediators can also be used for this purpose.

### Pharmacodynamic analysis of E5564 in vivo

As is shown in Figure 1 and Table 1, delivery of 300 ng/kg of LPS into beagle dogs ("control" in Figure 1) elicits a strong, reproducible response, as measured by levels of plasma TNF-α. Administration of 0.1 mg/kg E5564 is completely effective in blocking this LPS challenge when given at the same time as LPS (compare control to simultaneous administration in Figure 1). Similar results are obtained when LPS is given one hour after drug administration. Surprisingly, however, efficacy of E5564 decreased thereafter. That is, if this same challenge of endotoxin is administered three hours after administration of the E5564 dose (E5564 3 hours before LPS administration), activity of the endotoxin antagonist is greatly decreased, to about 48% of its original activity. This short activity lifetime (i.e., pharmacodynamic half-life) is an unexpected result, as the pharmacokinetic half-life of E5564 is extremely long in comparison (see below). Thus, while the unmodified (unmetabolized) drug appears to remain in circulation for a relatively long period of time, it loses activity. Because of this unexpected discovery, we have chosen to administer E5564 by infusion.

### In vivo pharmacokinetic analysis of E5564 after bolus injection

As is shown in Figures 3 and 4 and in Tables 2 and 3, E5564 demonstrates a relatively long half-life in blood after injection either as a bolus (Figure 3 and Table 2) or after infusion (Figure 4 and Table 3). This analysis of E5564 levels indicates that E5564 remains in the blood (or plasma), and is not rapidly removed or "cleared" by organs such as the liver, lungs, or kidneys, etc. This long-term presence of unmodified E5564 in blood initially led us to believe that active drug was likely present for very long periods of time after cessation of drug administration. As subsequent experiments demonstrated, this initial, reasonable supposition turned out to be wrong.

### In vivo pharmacokinetic analysis of E5564 during infusion

To assess the activity of E5564 over multiple time points from a single treated animal, we employed an *ex vivo* assay, as is mentioned above, to test for active drug in samples of blood drawn from a treated animal. Samples of blood were drawn from beagle dogs infused with E5564 over a period of 24 hours. One dog of each sex was tested under each of two dose regimens: a low dose of 0.24 mg/kg/hr and a high dose of 2.4 mg/kg/hr.

Blood samples were taken from these dogs at predose, 4 hours after initiation of infusion, and 24 hours after initiation of infusion. The blood samples were challenged with 0, 1, 10, or 100 ng/ml LPS, and then incubated for three hours. The samples were then analyzed for activation by LPS, using induction of cytokine response as a readout. As is shown in Figures 5-7, E5564 dose-dependently inhibited the LPS response in a time-dependent fashion.

### Inhibition of LPS-inducedIL-6 release in ex vivo blood samples by intravenous infusion of E5564in beagle dogs

### Effect of infusion of E5564 at 0.24 mg/kg/hr

As is shown in Figures 5-7, E5564 infused at 0.24 mg/kg/hr inhibited LPS response in *ex vivo* blood samples when compared to predose levels. Differences in inhibitory activity of E5564 were seen with respect to the amount of LPS added. Nearly complete (>98%) inhibition of response to 1 ng/ml LPS was seen with blood samples tested *ex vivo* at 4 hours after beginning infusion (see Figure 5). At the end of infusion, inhibition of 1 ng/ml LPS challenge was complete in samples obtained from both low dose LPS treated dogs. When blood samples were challenged with 10 ng/ml LPS, 29 to 70% inhibition was observed at 4 hours (Figure 5), and ∼85% inhibition was observed at the end of infusion. Challenges using 100 ng/ml LPS were only poorly inhibited by this rate of drug infusion; maximum inhibition was 34-52% (Figure 7) at the end of infusion.

### Effect of infusion of E5564 ay 2.4 mg/kg/hr

As is shown in Figure 5, samples taken 4 hours after beginning infusion of 2.4 mg/kg/hr E5564 exhibited complete inhibition of response to 1 ng/ml LPS, as compared to samples taken prior to beginning infusion, and nearly completely inhibited challenges of 10 and 100 ng/ml LPS. At the end of infusion (24 hours), inhibition was complete for the 1 and 10 ng/ml LPS challenges, and was 90% for the higher dose challenge of 100 ng/ml LPS.

These results show that infusion of E5564 at a dose of either 0.24 mg/kg/hr or 2.4 mg/kg/hr inhibits LPS response in blood over the period of infusion. Inhibition of LPS response is dose dependent for both E5564 and for the concentration of LPS used as challenge.

**Table 1. Pharmacodynamic analysis of E5564 after single bolus intravenous injection to dogs.**

| | Response to LPS¹ | | | | |
|---|---|---|---|---|---|
| Treatment | TNF-α at 1 hr² | TNF-α at . 2 hr² | Sum of TNF-α (AUC) | Response to LPS (% of control) | Inhibition of LPS response |
| None (LPS only) | 2369 ± 187 | 590 ±108 | 2959 | 100 | N/A |
| E5564 simultaneously with LPS | 0 | 0 | 0 | 0 | 100 |
| E5564 one hour before LPS | 0 | 0 | 0 | 0 | 100 |
| E5564 three hours before LPS | 1353 ± 340 | 190 ± 113 | 1543 | 52 | 48 |

| | | | | | |
|---|---|---|---|---|---|
| ¹Response to LPS was measured in groups of four beagle dogs for each treatment. ²Plasma levels of TNF-α induced at one and two hours after LPS administration. All TNF-α measured in units/ml. | | | | | |

**Table 2. Pharmacokinetic parameters for E5564 in plasma after single bolus intravenous injection to dogs.**

| | Dose | | |
|---|---|---|---|
| Parameter | 0.1 mg/kg dose | 0.3mg/kg | 1 mg/kg |
| T½ (hours) | 41.7 ± 2.7 | 50.4 ± 2.2 | 46.4 ± 2.7 |
| AUC (ng-hr/ml) | 71825.5 ± 1981 | 270897.2 ± 28260.8 | 743544.6 ± 90918.6 |

**Table 3. Pharmacokinetic parameters for E5564 in plasma after 72 hours intravenous infusion into dogs.**

| | | Parameter | | |
|---|---|---|---|---|
| Dose | Sex | C₇₂ₕᵣ (µg/ml) | AUC_{0-144 hr} (µg-hr/ml)¹ | T½ (hr)² |
| 0.1 mg/kg | Male | 13.07 ± 1.22 | 1069 ± 139 | 38.4 ± 4.5 |
| | Female | 10.35 ± 0.93 | 790 ± 51 | 40.9 ± 9.5 |
| 0.3 mg/kg | Male | 41.29 ± 7.18 | 3309 ± 485 | 39.9 ± 11.2 |
| | Female | 32.40 ± 5.72 | 2616 ± 737 | 38.0 ± 11.6 |
| I mg/kg | Male | 366 ± 69.3 | 27114 ± 5737 | 35,1 ± 8.7 |
| | Female | 393.9 ± 14.2 | 29642 ± 1514 | 32.0 ± 2.8 |

| | | | | |
|---|---|---|---|---|
| ¹Rounded to the nearest whole number ²T½ after end of infusion | | | | |

### Appendix

### (1) In vivo assays

### (1.1) Reagents

E5564 was synthesized by Eisai Research Institute of Boston, Andover, MA, USA. E5564 drug product was manufactured at the Eisai Preclinical Laboratory (Tsukuba, Japan) by dissolving 35.4 mg of drug substance in 52.1 ml 0.01N NaOH, stirring for one hour at room temperature, and diluting into phosphate-buffered lactose. After adjusting the pH to 7.3 and diluting to a final concentration of 0.1 mg/ml E5564, the solution was filter-sterilized and lyophilized.

The formulation of drug product in 1 ml vials is shown below.

| Material | amount |
|---|---|
| E5564 | 100 µg |
| NaH₂PO₄-4H₂O | 0.35 mg |
| NaOH | 0.06 mg |
| Lactose hydrous | 100 mg |
| Na₂HPO₄-7H₂O | 0.45 mg |
| sterile water | 1 ml |

*Escherichia coli* LPS (Serotype 0111:B4; phenol extracted, Cat. # L-2630) was purchased from Sigma Chem. Co. Ltd., St. Louis, MO, USA. Lyophylized E5564 was solubilized in 5 ml of sterile water (Otsuka Pharm. Co. Ltd., Tokyo, Japan). LPS was weighed to an accuracy of 1/10 mg and solubilized in 5% glucose (Otsuka Pharm. Co. Ltd., Tokyo, Japan). The LPS solution was sonicated with a bath-type sonicator for 15 minutes after which aliquots were immediately prepared and stored at -20°C. Prior to use, the solution was sonicated for one or two minutes, and then dilutions were prepared in 5% glucose.

### (1.2) Animals

Nine month-old beagles were obtained from Kawashima-shoji Co. Ltd., Gifu, Japan) and housed in stainless steel wire cages (W 800 mm X D 680 mm X H 680 mm; one dog per cage) in a room with a constant temperature of 20-24°C, humidity of 45-65%, and 12 hour light-dark cycle. The animals were provided with pellet food (DS, Oriental Yeast Co., Tokyo, Japan) and water *ad libitum.* LPS endotoxin (300 ng/0.1 ml/kg) was injected into the vein of the right foreleg at a rate of 1-2 ml/min, and E5564 was injected into the vein of the left foreleg at a rate of 10-20 ml/min.

### (1.3) Blood collection and treatment

Immediately before or 1, 2, or 4 hours after intravenous injection of LPS and E5564, 1.5 ml of blood was drawn from the left cephalic vein. One milliliter was transferred into a tube containing 10 U of heparin (Mochida Pharm. Co. Ltd., Tokyo, Japan), centrifuged (2000 x g, 5 minutes, 4°C), and the plasma was used for bioassays for TNF-α and IL-6.

### (1.4) TNF Bioassay

Aliquots of the plasma were tested for TNF in a bioassay based on the TNF-dependent cell death of L-P3 cells in the presence of actinomycin D. The L-P3 cell line is more sensitive to TNF-induced cell death than the L-929 cell line that is more commonly used.

L-P3 cells were cultured in RPMI 1640 medium containing 10% heat inactivated fetal calf serum, 100 U/ml penicillin, and 100 µg/ml streptomycin. Plasma samples to be assayed were diluted 5-100 fold, and 0.1 ml of each was serially diluted into 96-well culture plates. 7 × 10⁴ L-P3 cells in 100 µl medium containing 1 µg/ml actinomycin D mannitol (Sigma Chem. Co. Ltd., St. Louis, MO, USA) were added to each well containing the plasma samples and incubated for 15 hours at 37°C in 5% CO₂, TNF-induced cell toxicity was measured using methylene blue as follows. Wells were washed with water at least 5 times to remove dead cells, after which cells were fixed with 50 µl glutaraldehyde and stained with 0.1 ml of a 0.05% methylene blue solution in water for 15 minutes. Excess methylene blue was removed by washing at least 5 times, after which the plate was dried. Methylene blue was then re-extracted from cells by addition of 0.2 ml of 0.33 N HCI to each well, and absorbance was read with dual wavelengths of λ1⁴⁰⁵ and λ2⁶⁶⁰ nm on a microplate reader (ImmunoReader NJ-2000; Japan InterMed Co. Ltd., Tokyo, Japan).

### (1.5) IL-6 Bioassay

Aliquots of the plasma were tested for IL-6 activity by measuring IL-6-dependent proliferation of the mouse-derived lymphoma cell line, B9. Cells were cultured in RPMI 1640 medium containing 10% heat inactivated fetal calf serum, 50 µM 2-mercaptoethanol, 100 U/ml penicillin, 100 µg/ml streptomycin, and 2 mM glutamate. Plasma samples diluted ten-fold or 500 pg/ml of IL-6 standard (human recombinant IL-6; Genzyme Corporation, Boston, MA) were added to each well of a 96-well culture plate and then diluted serially. 1.5 × 10³ B9 cells in 50 µl medium were added to each well and the plates were incubated for three days at 37°C in 5% CO₂.

B9 cell proliferation was measured by the MTT (3-[4,5-Dimethylthiazol-2-yl]-2,5-diphenyltetrazolium bromide; Sigma Chem. Co., St. Louis, MO, USA) staining method. Twenty microliters of 6 mg/ml of MTT in Dulbecco phosphate-buffered saline were added to each well and the plates were incubated for 3 hours at 37°C in 5% CO₂. Next, 100 µl/well of 10% SDS (sodium dodecyl sulfate; Nacalai Tesque Co. Ltd., Kyoto, Japan) in 1 mM NH₄OH was added and the cells were then solubilized overnight. Absorbance of each well was read by a plate reader (Model 3550, Bio-Rad Labs, Richmond, CA, USA) with dual wavelengths of λ1⁵⁴⁰ and λ2⁶⁶⁰ nm. One unit/ml of human IL-6 is equivalent to 100 pg/ml.

### (2) Ex vivo assays

### (2.1) Reagents

LPS from *Escherichia coli* (0111:B4) was purchased from List Biologicals (Campbell, CA). LPS was dissolved in sterile water at 1 mg/ml and stored at -20°C. Prior to use, LPS was sonicated in a bath sonicator (VW-380; Heat Systems-Ultrasonics Inc., Farmingdale, NY) for 1-2 minutes immediately before use and diluted into Ca²⁺, Mg²⁺ free Hanks balanced salt solution (HBSS; Sigma).

### (2.2) Origin of samples and study design

Dogs were treated with E5564 (0.24 or 2.4 mg/kg/hr) dissolved in a mixture of placebo solution (10% lactose monohydrate, 0.045% Na₂HPO₄-7H₂O, 0.035% Na₂HPO₄-7H₂O, 0.006% NaOH; pH 7.4±0.3) and 5% dextrose (1:4) by intravenous infusion *via* indwelling catheter for 24 hours at a rate of 2 mg/kg/hr. The study design is shown in the following table:

| Group | Dose Level | Animal Numbers | |
|---|---|---|---|
| No. | (mg/kg/hr) | Male | Female |
| 1 | 0.24 | 101 | 151 |
| 2 | 2.4 | 201 | 251 |

### (2.3) Analysis of E5564 activity in dog whole blood

Prior to and during infusion of E5564, blood was drawn into heparinized syringes, and either aseptically reduced to plasma by centrifugation and frozen to
-80 ° C (for time zero samples), or incubated with the indicated concentrations of LPS for three hours. Plasma was then prepared and immediately frozen at - 80°C. Samples were stored at -80°C until assay.

### (2.4) Bioassay for IL-6

B9 cells were the gift of Dr. Mary Rodrick (Beth Israel Deaconess Hospital, Boston, MA). They were grown in Iscove's DMEM medium containing 5% fetal bovine serum (FBS), 20 mM 2-mercaptoethanol, 2 mM L-glutamine, and 100 U/ml penicillin/streptomycin. For maintenance of growth, these cells were kept in growth media containing 50 U/ml (or 1 ng/ml) recombinant human IL-6 (Genzyme). For growth dependence by IL-6 (IL-6 bioassay), B9 cells were washed three times in assay media and counted, cell concentration was adjusted to 4 x 10⁵/ml (2 × 10⁴/50 µl) in assay media, and 50 µl of media was added to each well of a 96-well tissue culture plate.

To the above-described cell suspension, 50 µl of standard or sample was added to each well, and the cells cultured for 68-72 hours at 37°C/5% CO₂. Dog plasma samples were added to the assay at a 1:20 dilution (10 µl + 190 µl) in assay media (in duplicate), then serially diluted 1:4 (to a final dilution of 1:327,680) in 96-well microtitre plates. Fifty microliters of each dilution were then transferred to an appropriately labeled assay microtitre plate. Standard curves were prepared (2-4 rows/plate, depending on plate space) using human rIL-6 as a standard (10 µg/ml), diluted 1:100, and then diluted another 1:10 to 10 ng/ml. Two hundred microliters of this dilution were added to a dilution plate, then each was serially diluted 1:4, and 2 blank wells received 50 µlassay media only. After the culture period, actively metabolizing cells were quantitated by adding 10 µlof a 5 mg/ml solution of MTT (3-[4,5-Dimethylthiazol-2-yl]-2,5-diphenyltetrazolium bromide) in sterile PBS to each well. Plates were incubated 4-5 hours at 37°C, acid-isopropanol (150 µl 40 mM HCL in isopropanol) was added to each well, plates were incubated for 1 hour at 37 ° C, followed by repeated trituration to solubilize crystals, and absorbance was read at 540 and 690 nm (background absorbance). IL-6 concentration was determined by calculation of a linear relationship for response to IL-6 standards that yielded the greatest dose-response region of the standard curve. (In general, this range is between 0.016 and 0.063 ng/ml IL-6, yielding net absorbances of ∼0.3 to 0.4 for the low dose and ∼0.8 to 1.0 for the high dose.) Only absorbances that fell between the above values for standards (or ± 0.05 AU) were used to calculate IL-6 by interpolation from the linear curve drawn by the Four Parameter Curve Fit program (Delta Soft) through the standard points.

### (2.5) Induction of IL-6 by LPSchallenge in ex vivo blood_samples

To obtain baseline values for LPS stimulation, samples of blood were drawn at approximately one hour prior to beginning administration (predose). While we did not extensively analyze the dose response relationship of dog blood to LPS, we used 1, 10, and 100 ng/ml LPS to ensure that a measurable response could be generated. Responses to LPS in these samples resulted in 6,000 pg/ml IL-6 to as high as 40,000 pg/ml IL-6 in response to 100 ng/ml LPS in the four dogs. Some samples (particularly from the two female dogs) demonstrated a more graded response to the three different concentrations of LPS. However, all LPS-challenged predose samples generated between 3,000 pg/ml IL-6 and 32,000 pg/ml IL-6. Blood from the male beagles responded more vigorously than blood from the female dogs.

## Claims

1. Use of a compound having the formula α-D-Glucopyranose, 3-*O*-decyl-2-deoxy-6-*O*-[2-deoxy-3-O-[(3R)-3-methoxydecyl]-6-*O*-methyl-2-[[(11 Z)-1-oxo-11-octadecenyl]amino]-4-*O*-phosphono-β-D-glucopyranosyl]-2-[(1,3-dioxotetradecyl)amino]-1-(dihydrogen phosphate) for the preparation of a pharmaceutical composition for treating a patient suffering from a medical condition amenable to treatment with said compound, wherein the pharmaceutical composition is to be administered to said patient by intravenous infusion over a period of 12-100 hours, the medical condition being HIV infection.

2. The use of claim 1, wherein infusion is carried out over a period of 60-80 hours.

3. The use of claim 2, wherein infusion is carried out over a period of 72 hours.

4. The use of claim 1, wherein the infusion/dosage rate is 0.001-0.5 mg/kg body weight/hour.

5. The use of claim 4, wherein the infusion/dosage rate is 0.01-0.2 mg/kg body weight/hour.

6. The use of claim 5, wherein the infusion/dosage rate is 0.03-0.1 mg/kg body weight/hour.

7. The use of claim 1, wherein said infusion is preceded by a bolus injection of said compound.

8. The use of claim 7, wherein said bolus injection is at a dosage of 0.001-0.5 mg/kg body weight.

9. The use of claim 1, wherein the total amount of said compound administered to the patient is 50-600 mg

10. The use of claim 9, wherein the amount of said compound administered is 150-500 mg, over a period of 60-80 hours.
